(19) Europäisches Patentamt
European Patent Office
Office européen des brevets

(11) **EP 1 971 258 B1**

(12) **FASCICULE DE BREVET EUROPEEN**

(45) Date de publication et mention
de la délivrance du brevet:
**22.06.2022  Bulletin 2022/25**

(21) Numéro de dépôt: **07712645.6**

(22) Date de dépôt: **12.01.2007**

(51) Classification Internationale des Brevets (IPC):
***A61B 5/00*** *(2006.01)*

(52) Classification Coopérative des Brevets (CPC):
**A61B 5/0073;** A61B 5/0091

(86) Numéro de dépôt international:
**PCT/FR2007/000054**

(87) Numéro de publication internationale:
**WO 2007/080326 (19.07.2007 Gazette 2007/29)**

(54) **PROCEDE DE RECONSTRUCTION D'UNE IMAGE TRIDIMENSIONNELLE DE TOMOGRAPHIE OPTIQUE DE FLUORESCENCE PAR DOUBLE MESURE**

VERFAHREN ZUR WIEDERHERSTELLUNG EINES 3D-BILDES EINER OPTISCHEN FLUORESZENZ-TOMOGRAPHIE DURCH DOPPELMESSUNG

METHOD FOR RECONSTRUCTING A FLUORESCENCE OPTICAL TOMOGRAPHY THREE-DIMENSIONAL IMAGE BY DOUBLE MEASUREMENT

(84) Etats contractants désignés:
**AT BE BG CH CY CZ DE DK EE ES FI FR GB GR HU IE IS IT LI LT LU LV MC NL PL PT RO SE SI SK TR**

(30) Priorité:  **12.01.2006  FR 0600286**

(43) Date de publication de la demande:
**24.09.2008  Bulletin 2008/39**

(73) Titulaire: **Commissariat à l'Énergie Atomique et aux Énergies Alternatives**
**75015 Paris (FR)**

(72) Inventeurs:
• **KOENIG, Anne**
**F-38410 Saint Martin d'Uriage (FR)**
• **HERVE, Lionel**
**F-69100 Villeurbanne (FR)**

(74) Mandataire: **INNOV-GROUP**
**310, avenue Berthelot**
**69372 Lyon Cedex 08 (FR)**

(56) Documents cités:
EP-A1- 0 385 608     WO-A-99/20997
US-A- 4 945 239      US-A- 5 628 314
US-A- 5 787 887      US-A- 5 876 339

• POGUE B W ET AL: "COMPARISION OF IMAGING GEOMETRIES FOR DIFFUSE OPTICAL TOMOGRAPHY OF TISSUE" OPTICS EXPRESS, OPTICAL SOCIETY OF AMERICA, WASHINGTON, DC,, US, vol. 4, no. 8, 12 avril 1999 (1999-04-12), pages 270-286, XP000980440 ISSN: 1094-4087 cité dans la demande
• S R ARRIDGE: "Optical tomography in medical imaging" INVERSE PROBLEMS, no. 15, 1999, pages R41-R93, XP002404811
• NTZIACHRISTOS VASILIS ET AL: "Charge-coupled-device based scanner for tomography of fluorescent near-infrared probes in turbid media" MEDICAL PHYSICS, AIP, MELVILLE, NY, US, vol. 29, no. 5, mai 2002 (2002-05), pages 803-809, XP012011792 ISSN: 0094-2405

EP 1 971 258 B1

**Description**

**Domaine technique de l'invention**

L'invention concerne un procédé de reconstruction d'une image tridimensionnelle de tomographie optique par fluorescence pour examiner un objet, en forme de plaque ayant une première face et une seconde face opposée et comportant des fluorophores, procédé comportant :

- une première étape d'éclairement séquentiel de la première face de l'objet avec une lumière d'excitation des fluorophores à partir d'une pluralité de points d'illumination, la première étape d'éclairement comportant une succession de phases d'éclairement à partir d'un point d'illumination associé à chaque phase, et
- une première étape d'acquisition séquentielle d'une première série d'images, une image de ladite série étant formée, lors de chaque phase d'éclairement de la première face, par un détecteur comportant une pluralité de points de détection détectant simultanément une lumière émise par la seconde face de l'objet.

**État de la technique**

La tomographie optique de fluorescence (fDOT) consiste à déterminer la répartition tridimensionnelle de fluorophores dans un objet comportant un milieu homogène diffusant. Les fluorophores peuvent être fonctionnalisés pour cibler des cellules tumorales et, ainsi, marquer des cellules cancéreuses. L'article "Comparison of imaging geometries for diffuse optical tomography of tissue" de B.W.Pogue et al. (Optics Express Vol.4, No.8, 1999) compare différentes géométries de tomographie optique diffusive (DOT).

Lorsque l'objet est en forme de plaque ou tranche (géométrie de type « slab » en anglais), une face avant de l'objet est éclairée par une lumière d'excitation dont le spectre correspond au fluorophore. La lumière d'excitation est alors déplacée point par point sur la surface de l'objet et une caméra prélève une série d'images de la lumière de fluorescence qui est émise par la face arrière de l'objet. Cette géométrie, dans laquelle les faces avant et arrière opposées constituent deux plans sensiblement parallèles, est fréquemment utilisée en mammographie et imagerie du petit animal.

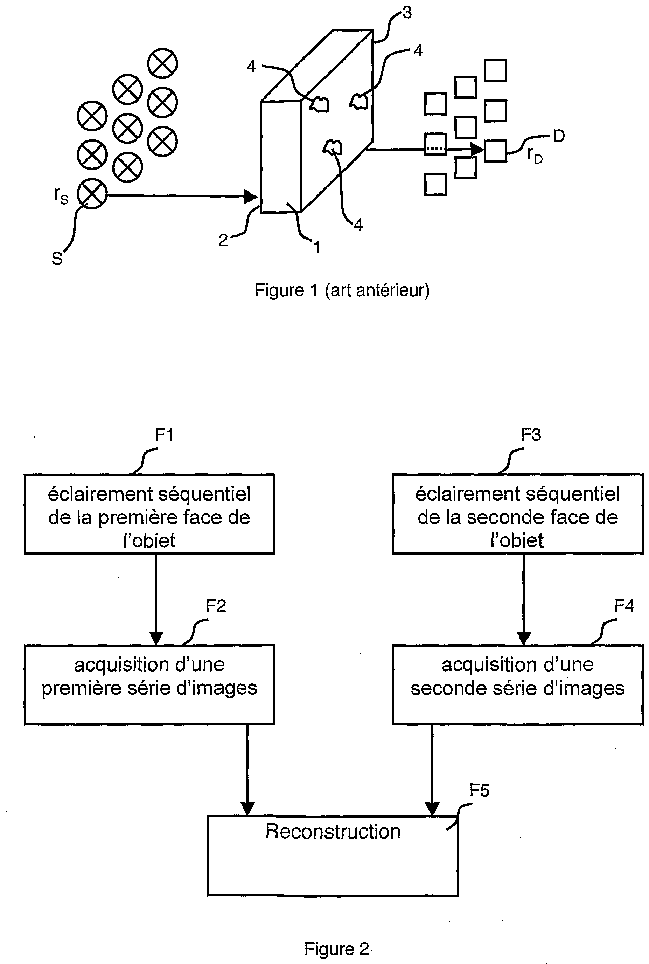

Comme représenté très schématiquement sur la figure 1, l'objet 1 est éclairé par un jeu de sources lumineuses S d'une longueur d'onde d'excitation $\lambda$ex placées dans un même plan vis-à-vis d'une première face 2 de l'objet 1. La lumière transmise à la longueur d'onde d'excitation $\lambda$ex et la lumière émise par les fluorophores 4 avec une longueur d'onde d'émission $\lambda$em sont détectées par un jeu de détecteurs D disposé dans un même plan vis-à-vis d'une seconde face 3 de l'objet 1, opposée à la première face 2. En pratique, le jeu de sources peut être remplacé par un laser dont le faisceau se déplace sur la première face 2 de l'objet 1. L'objet est alors éclairé séquentiellement à partir de chacun des points d'illumination constitués par chacune des sources ou chacune des positions du laser.

Les longueurs d'onde d'excitation $\lambda$ex et d'émission $\lambda$em sont détectées séparément par l'intermédiaire de filtres optiques. Ainsi, on distingue les signaux Uex(s,d) détectés à la longueur d'onde d'excitation $\lambda$ex et les signaux Uem(s,d) détectés à la longueur d'onde d'émission $\lambda$em, s et d étant les indices identifiant respectivement la source S et le détecteur D correspondant au signal U(s,d), c'est-à-dire la source S et le détecteur D activés pour l'acquisition du signal U(s,d). Une image ou, plus généralement, des signaux représentatifs d'une image, sont fournis par le jeu de détecteurs lors de chaque phase d'éclairement par un des points d'illumination.

Le traitement des signaux Uex(s,d) et Uem(s,d) permet, à partir d'une série d'images acquises séquentiellement et correspondant respectivement à l'éclairement de l'objet par l'un des points d'illumination, de reconstruire une image tridimensionnelle de la répartition des fluorophores 4 dans l'objet 1. Les algorithmes pour résoudre ce problème sont connus et décrits, par exemple, dans l'article « Optical tomography in medical imaging » de S.R.Arridge (Inverse Problems 15, R41-R93, 1999). Le problème est notamment résolu à partir de l'équation de diffusion, établie à partir de l'équation de transfert radiatif. Chaque source S génère dans le milieu une onde diffusive ayant la longueur d'onde $\lambda$ex. L'onde diffusive se propage dans le milieu et une partie de l'énergie de l'onde diffusive excite les fluorophores 4 qui peuvent être considérés comme des sources secondaires réémettant un rayonnement à la longueur d'onde d'émission $\lambda$em.

L'équation de diffusion est résolue par l'intermédiaire de fonctions de Green dans une géométrie de plans parallèles, ce qui permet d'obtenir une expression analytique pour la propagation de l'onde diffusive dans le milieu.

En ce qui concerne le signal de fluorescence, on considère que chaque fluorophore 4 présente un paramètre de conversion de photons correspondant à la fraction de l'énergie incidente qui est réémise avec la longueur d'onde d'émission $\lambda$em. De manière plus générale, on peut associer à chaque élément de volume M (voxel) de l'objet 1 un paramètre de conversion X(m), où m est l'indice de maillage identifiant les voxels M qui sont définis selon un maillage quelconque du volume de l'objet 1. On note G(m,d) la fonction de Green correspondant à la propagation de lumière entre un voxel M et un détecteur D. De plus, on note G(s,m) la fonction de Green correspondant à la propagation de lumière entre une source S et un voxel M. Le flux incident dans le voxel M et correspondant à la source S est proportionnel à Q(s)G(s,m), où Q(s) est le flux émis par la source S. Le flux réémis par le voxel M avec la longueur d'onde d'émission λem est donné par Q(s)G(s,m)X(m). La contribution du voxel M excité par la source S, au signal détecté par le détecteur D est donc proportionnelle à Q(s)G(s,m)X(m)G(m,d). En considérant l'ensemble du volume de l'objet, le signal Uem(s,d) est proportionnel à la somme des contributions de tous les voxels M, c'est-à-dire à l'expression Q(s)Σm(G(s,m)X(m)G(m,d)). Lorsque les flux Q(s) émis par les sources S sont constants et égaux pour les différentes sources, on peut incorporer les différentes constantes dans les paramètres de conversion X(m) et on obtient :

$$U^{em}_{s,d} = \sum_m G_{s,m} X_m G_{m,d} \quad (1).$$

On obtient ainsi un système linéaire d'équations reliant les mesures des détecteurs aux paramètres de conversion X(m) inconnus et recherchés.

L'acquisition d'un nombre suffisant de mesures permet ainsi de reconstruire la répartition des fluorophores 4 dans l'objet 1. La reconstruction proprement dite est, par exemple, effectuée au moyen d'un algorithme itératif de type ART (Algebraic Reconstruction Technique) qui minimise l'erreur entre les mesures expérimentales et le résultat analytique calculé.

L'algorithme ART minimise l'erreur

$$\left\| \frac{U^{em}_{s,d}}{U^{ex}_{s,d}} - \frac{\sum_m G_{s,m} X_m G_{m,d}}{U^{ex}_{s,d}} \right\|^2 = \left\| Y_{mes} - WX_m \right\|^2 (2),$$

avec W=G(s,m)G(m,d)/Uex(s,d).

La lumière d'excitation de la fluorescence et la lumière de fluorescence sont habituellement situées dans la bande spectrale du proche infrarouge, puisque le pouvoir de pénétration de cette lumière est relativement important dans des tissus biologiques. Le coefficient d'extinction étant par exemple de 2,5cm-1, l'intensité de lumière est réduite par un facteur 12 pour chaque centimètre pénétré, ce qui exclut l'étude dans une profondeur supérieure à 10 cm.

Etant donné que l'objet présente une certaine épaisseur, les fluorophores ne sont pas tous à la même distance ni de la source de lumière, ni des détecteurs, ce qui entraîne une perte de précision dans la reconstruction de l'image. En effet, les zones de l'objet situées près du plan des sources de lumière sont imagées avec une qualité moindre que celles qui sont près du plan des détecteurs, comme indiqué dans l'article « Effects of sampling and limited data in optical tomography » de V.A.Markel et al. (Applied Physics Letters Vol. 81, No.7, 2002).

L'article « Dual-projection optical diffusion tomography » de V.A.Markel et al. (Optics letters Vol. 29, No.17, 2004) propose d'utiliser deux projections orthogonales pour améliorer la résolution en profondeur par rapport à la résolution transverse, ce qui ne permet pas d'examiner des objets sous forme de tranche dont l'épaisseur est de l'ordre du centimètre et dont la largeur et la hauteur sont nettement supérieures au centimètre. L'article « Singular-value analysis and optimization of experimental parameters in fluorescence molecular tomography » de E.E.Graves et al. (J. Opt. Soc. Am. Vol. 21, No.2, 2004) étudie les distributions optimales de sources de lumière et de détecteurs pour la géométrie en plans parallèles.

Par ailleurs, dans le cas où l'ensemble de détecteurs est constitué par une caméra, le nombre de points éclairés individuellement est souvent beaucoup plus petit que le nombre de pixels de la caméra. Cette différence de résolution de part et d'autre de l'objet mène à une différence de précision dans l'épaisseur du volume reconstruit.

**Objet de l'invention**

L'invention a pour but de remédier à ces inconvénients et, en particulier, d'améliorer la résolution de la reconstruction d'une image tridimensionnelle de la répartition de fluorophores dans un milieu diffusant ayant la forme d'une plaque.

Selon l'invention ce but est atteint par un procédé selon les revendications annexées.

**Description sommaire des dessins**

D'autres avantages et caractéristiques ressortiront plus clairement de la description qui va suivre de modes particuliers de réalisation de l'invention donnés à titre d'exemples non limitatifs et représentés aux dessins annexés, dans lesquels :

La figure 1 illustre un procédé de reconstruction selon l'art antérieur.

La figure 2 montre schématiquement différentes étapes d'un procédé de reconstruction selon l'invention.

Les figures 3 et 4 illustrent un mode de réalisation particulier du procédé selon l'invention.

**Description de modes particuliers de réalisation**

L'invention concerne une géométrie en plans parallèles, l'objet 1 ayant, typiquement, une épaisseur comprise entre 10 et 15mm. Un jeu de sources S de lumière constituant une série de points discrets d'illumination de l'objet est, de préférence, constitué par une source laser que l'on déplace dans un plan parallèle à l'objet selon deux directions orthogonales pour quadriller l'objet 1. Typiquement, le laser décrit, dans ce plan, une grille de 11 par 15 positions avec un pas de 3mm. Selon la précision à obtenir, le pas peut être réduit et selon la taille de la zone à observer, le nombre de points peut être

**Description de modes particuliers de réalisation**

L'invention concerne une géométrie en plans parallèles, l'objet 1 ayant, typiquement, une épaisseur comprise entre 10 et 15mm. Un jeu de sources S de lumière constituant une série de points discrets d'illumination de l'objet est, de préférence, constitué par une source laser que l'on déplace dans un plan parallèle à l'objet selon deux directions orthogonales pour quadriller l'objet 1. Typiquement, le laser décrit, dans ce plan, une grille de 11 par 15 positions avec un pas de 3mm. Selon la précision à obtenir, le pas peut être réduit et selon la taille de la zone à observer, le nombre de points peut être augmenté (par exemple de l'ordre de 30 points ou plus dans les deux directions).

L'ensemble de détecteurs est, de préférence, constitué par une caméra CCD couplée à une lentille permettant de détecter une image de l'objet dans un plan parallèle à la surface de l'objet 1. La caméra acquiert ainsi une image pour chaque position de la source laser. Plus précisément, lorsque l'objet est éclairé séquentiellement à partir des différents points d'illumination, la caméra fournit une série d'images correspondantes acquises séquentiellement.

La résolution intrinsèque de l'image est déterminée par la taille des pixels de détection et par le grandissement du système optique. Généralement, les images sont ensuite ré-échantillonnées pour limiter le nombre de points de détection et, ainsi, le temps de calcul. Typiquement, le ré-échantillonnage est effectué avec un pas quatre fois plus faible que celui des sources. Au final, on obtient ainsi 16 fois plus de points de détection que de points de source. Ainsi, plus particulièrement lorsqu'une caméra CCD pixellisée, comportant une matrice dense de points de détection, est utilisée, il existe une dissymétrie non négligeable entre la densité, relativement faible, des points d'illumination et la densité, plus élevée, des points de détection. Cette dissymétrie provoque une dissymétrie dans la résolution en profondeur de l'image reconstruite (selon un axe perpendiculaire au plan des faces de l'objet).

L'invention tire parti de cette dissymétrie pour améliorer la résolution de l'image tridimensionnelle finale.

Comme illustré à la figure 2, on effectue une première étape d'éclairement séquentiel de la première face 2 de l'objet 1 (F1) avec une lumière d'excitation des fluorophores ayant une longueur d'onde d'excitation $\lambda$ex. De manière connue, une première série d'images est acquise (F2), pendant une succession de phases d'éclairement, par détection d'une lumière émise par la seconde face 3 de l'objet 1, qui peut comporter la longueur d'onde d'excitation $\lambda$ex et la longueur d'onde d'émission $\lambda$em du fluorophore 4. Comme précédemment, une phase d'éclairement consiste à éclairer l'objet à partir d'un point d'illumination correspondant.

Le procédé de reconstruction d'image selon l'invention comporte une seconde étape d'éclairement séquentiel (F3), consistant à éclairer la seconde face 3 de l'objet 1 avec une lumière d'excitation des fluorophores ayant une longueur d'onde d'excitation $\lambda$ex. Comme précédemment, l'étape d'éclairement comporte une succession de phases d'éclairement consistant chacune à éclairer la seconde face de l'objet à partir d'un point d'illumination correspondant.

Une seconde étape d'acquisition séquentielle d'une seconde série d'images (F4) est associée à la seconde étape d'éclairement. La seconde étape d'acquisition séquentielle détecte, pendant la succession de phases d'éclairement correspondantes de la seconde face 3, la lumière émise par la première face 2 de l'objet 1, qui peut comporter la longueur d'onde d'excitation $\lambda$ex et la longueur d'onde d'émission $\lambda$em du fluorophore 4. La reconstruction (F5) de la répartition des fluorophores 4 dans l'objet 1 est effectuée par l'intermédiaire des première et seconde séries d'images.

Avantageusement, la lumière émise par la première face 2 et la lumière émise par la seconde face 3 sont filtrées afin de supprimer la lumière d'excitation. Ainsi, seule la longueur d'onde d'émission $\lambda$em du fluorophore 4 est détectée.

Ainsi, lors de la reconstruction, les différentes zones de l'objet 1 profitent d'une haute résolution grâce à une des deux étapes d'acquisition d'images et grâce au retournement de la mesure. La résolution n'est plus limitée du fait de l'épaisseur de l'objet 1.

Afin de reconstruire l'image de l'objet 1 par l'intermédiaire des première et seconde séries d'images, on complète le système linéaire précédemment décrit en utilisant deux jeux de matrices des fonctions de Green correspondant respectivement à la première et la seconde acquisition de séries d'images. A chaque itération de l'algorithme de type ART, au lieu de ne considérer qu'un système linéaire unique, on considère l'ensemble d'équations formé par les deux systèmes. La reconstruction d'une image tridimensionnelle de la répartition des fluorophores dans l'objet est ainsi effectuée en établissant deux systèmes linéaires d'équations à partir de deux jeux de matrices de fonctions de Green respectifs, que l'on fusionne en un seul.

Le premier et le second système linéaire d'équations, respectivement associés à la première et à la seconde acquisition de séries d'images (F2 et F4) peuvent s'écrire comme l'équation 1 ci-dessus. On peut définir un super indice i=i(s,d) de mesure qui englobe les indices s et d des sources de lumière et des détecteurs, par exemple conformément à la relation i=Nd.s+d, où Nd est le nombre des détecteurs. Avec W(i,m)=G(s,m)G(m,d), chaque système s'écrit alors :

$$U^{em}{}_i = \sum_m G_{s,m} X_m G_{m,d} \quad (3),$$

ce qui équivaut aux systèmes matriciels $U_1 = W_1 X$ et $U_2 = W_2 X$. On combine les deux systèmes correspondant respectivement aux deux acquisitions d'images en écrivant :

$$\begin{bmatrix} U_1 \\ U_2 \end{bmatrix} = \begin{bmatrix} W_1 \\ W_2 \end{bmatrix} \begin{bmatrix} X \end{bmatrix} ,$$

ou bien sous forme symbolique U = WX (4), où U est une matrice à une colonne et 2n lignes, W est une matrice à m colonnes et 2n lignes et X est une matrice à une colonne et m lignes, avec n=Nd.Ns (Ns est le nombre de sources). Comme défini ci-dessus, m est l'indice de maillage. On peut considérer les équations des deux systèmes séquentiellement ou les réarranger aléatoirement.

La mesure selon l'invention est notamment moins sensible aux mouvements des fluorophores 4 de l'objet 1, par exemple provoqués par un mouvement de l'objet 1 entier, comme dans le cas de la respiration. En effet, dans la géométrie en plans parallèles selon l'art antérieur, un fluorophore en mouvement disposé près des sources est moins bien reconstruit qu'un fluorophore en mouvement disposé près des détecteurs. Le fait d'utiliser l'acquisition de deux d'images par retournement permet non seulement de pallier à ce déséquilibre mais, de plus, d'améliorer la résolution sur toute l'épaisseur de l'objet 1.

Le traitement des première et seconde séries d'images permet d'obtenir un résultat amélioré par rapport à l'art antérieur, même si les première et seconde séries d'images sont traitées indépendamment. En effet, une zone proche de l'une des surfaces 2 ou 3 de l'objet 1 est ainsi toujours bien résolue grâce à celle des première et seconde séries d'images pour laquelle le détecteur est disposé près de la zone. Des simulations ont permis de montrer qu'il est encore possible d'améliorer la résolution du procédé de reconstruction par l'intermédiaire du traitement combiné des première et seconde séries d'images. Les deux systèmes linéaires d'équations sont alors, de préférence, combinés pour être résolus.

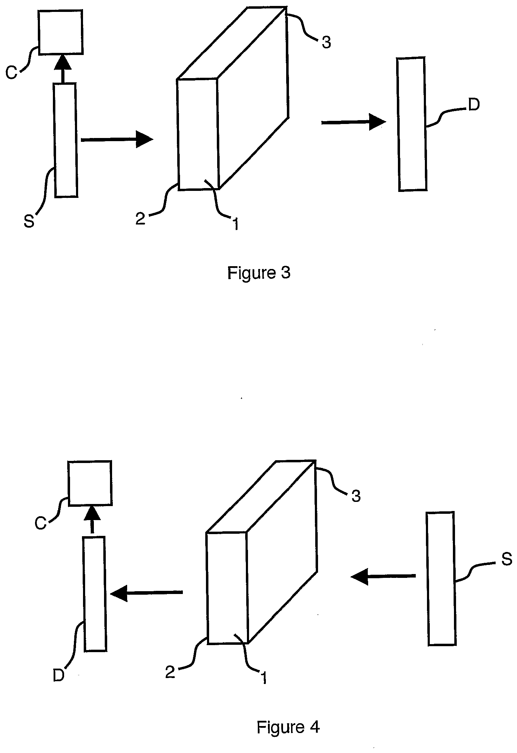

Le procédé peut être mise en œuvre par l'intermédiaire d'un dispositif comportant des sources lumineuses S pour l'éclairement de la première face 2 de l'objet 1 avec une lumière d'excitation λex des fluorophores 4, comme représenté à la figure 3. De plus, le dispositif comporte un détecteur D, de préférence une caméra pour l'acquisition d'une première série d'images par détection d'une lumière émise par la seconde face 3 de l'objet 1. Le détecteur D comporte, de préférence, un filtre pour éliminer la lumière d'excitation λex et il est connecté à une unité de calcul C. Le dispositif comporte également une source pour l'éclairement de la seconde face 3 de l'objet 1 avec une lumière d'excitation λex des fluorophores 4. Un second détecteur peut être utilisé pour l'acquisition d'une seconde série d'images par détection d'une lumière émise par la première face 2 de l'objet 1. Les deux détecteurs sont alors connectés à une unité de calcul C pour la reconstruction d'une image tridimensionnelle de la répartition des fluorophores 4 dans l'objet 1 par l'intermédiaire des première et seconde séries d'images.

Les première et seconde étapes d'éclairement (F1, F3) peuvent être effectuées avec une même source de lumière S et l'acquisition des deux séries d'image par un seul et même détecteur. Dans un premier mode de réalisation particulier, la source de lumière S et l'objet 1 sont déplacés relativement entre les première et seconde étapes d'éclairement. Dans l'exemple illustré aux figures 3 et 4, la source de lumière S et le détecteur D sont déplacés, tandis que l'objet 1 reste immobile. On peut également envisager de retourner l'objet 1. Dans un second mode de réalisation particulier, non représenté, la lumière d'excitation peut être dirigée sur les première (2) et seconde (3) faces de l'objet 1 par l'intermédiaire d'un ou de plusieurs miroirs.

Les première et seconde étapes d'acquisition d'image (F2, F4) peuvent ainsi être effectuées avec une même caméra. La caméra constituant le détecteur D est alors, par exemple, déplacée comme représenté sur les figures 3 et 4, ou bien l'objet est retourné comme décrit ci-dessus. Pour l'acquisition d'image également, on peut envisager de diriger la lumière provenant des première (2) et seconde (3) faces sur la caméra par l'intermédiaire d'un ou de plusieurs miroirs.

Il est à noter que l'acquisition de la seconde série d'images, obtenues par transmission à travers l'objet éclairé depuis sa seconde face, ne permet pas d'améliorer la précision de l'image finale si la densité (nombre et répartition) des points d'illumination et des points de détection est équivalente. En effet, cela correspondrait simplement à une transposition des matrices G(s,m).

Le procédé selon l'invention s'applique à tout objet en forme de plaque ou pouvant se ramener à une telle forme, en particulier un sein compressé. Par ailleurs le terme d'image utilisé pour désigner les images fournies par le détecteur couvre les signaux représentatifs d'une image.

## Revendications

**1.** Procédé de reconstruction d'une image tridimensionnelle de tomographie optique par fluorescence pour examiner un objet (1), en forme de plaque ayant une première face (2) et une seconde face (3) opposée et comportant des fluorophores (4), procédé comportant :

- une première étape d'éclairement séquentiel (F1) de la première face (2) de l'objet (1) avec une lumière d'excitation ($\lambda$ex) des fluorophores (4) à partir d'une pluralité de points d'illumination, la première étape d'éclairement comportant une succession de phases d'éclairement à partir d'un point d'illumination associé à chaque phase, et

- une première étape d'acquisition séquentielle d'une première série d'images (F2), une image de ladite série étant formée, lors de chaque phase d'éclairement de la première face, par un détecteur comportant une pluralité de points de détection détectant simultanément une lumière ($\lambda$ex, $\lambda$em) émise par la seconde face (3) de l'objet (1),

le procédé étant tel que la densité des points d'illumination étant inférieure à la densité des points de détection, le procédé comporte ensuite :

- une seconde étape d'éclairement (F3) séquentiel de la seconde face (3) de l'objet (1) avec une lumière d'excitation ($\lambda$ex) des fluorophores (4),

- une seconde étape d'acquisition séquentielle d'une seconde série d'images (F4) par détection d'une lumière ($\lambda$ex, $\lambda$em) émise par la première face (2) de l'objet (1) et

- la reconstruction (F5) de l'image tridimensionnelle de la répartition des fluorophores (4) dans l'objet (1) par l'intermédiaire des première et seconde séries d'images, ladite reconstruction (F5) étant effectuée en établissant deux systèmes linéaires d'équations établis en utilisant deux jeux de matrices des fonctions de Green correspondant respectivement à la première et la seconde acquisition de séries d'images, les deux systèmes linéaires d'équations étant combinés pour être résolus ;

le procédé étant **caractérisé en ce que** le nombre et la répartition des points d'illumination et des points de détection est différente entre la première étape d'éclairement et d'acquisition séquentiels et la deuxième étape d'éclairement et d'acquisition séquentiels

**2.** Procédé selon la revendication 1, **caractérisé en ce que** la lumière émise par la première face 2 et la lumière émise par la seconde face 3 sont filtrées pour supprimer la lumière d'excitation ($\lambda$ex) des fluorophores (4).

**3.** Procédé selon l'une quelconque des revendications 1 et 2, **caractérisé en ce que** les première et seconde étapes d'éclairement (F1, F3) sont effectuées avec une même source de lumière (S), la source de lumière (S) et l'objet (1) étant déplacés relativement entre les première et seconde étapes d'éclairement (F1, F3).

**4.** Procédé selon l'une quelconque des revendications 1 et 2, **caractérisé en ce que** les première et seconde étapes d'éclairement (F1, F3) sont effectuées avec une même source de lumière (S), la lumière d'excitation ($\lambda$ex) étant dirigée sur les première (2) et seconde (3) faces par l'intermédiaire d'au moins un miroir.

**5.** Procédé selon l'une quelconque des revendications 1 à 4, **caractérisé en ce que** les première et seconde étapes d'acquisition de séries d'image (F2, F4) sont effectuées avec une même caméra (D), la caméra (D) et l'objet (1) étant déplacés relativement entre les première et seconde étapes d'acquisition d'image (F2, F4).

**6.** Procédé selon l'une quelconque des revendications 1 à 4, **caractérisé en ce que** les première et seconde étapes d'acquisition de séries d'image (F2, F4) sont effectuées avec une même caméra (D), la lumière provenant des première (2) et seconde (3) faces étant dirigée sur la caméra (D) par l'intermédiaire d'au moins un miroir.

**7.** Procédé selon l'une quelconque des revendications 1 et 2, **caractérisé en ce que** l'objet (1) est retourné entre les première et seconde étapes d'éclairement.

**Patentansprüche**

**1.** Verfahren zur Rekonstruktion eines dreidimensionalen Bildes der optischen Fluoreszenztomographie, um einen plattenförmigen Gegenstand (1) zu untersuchen, welcher eine erste Fläche (2) und eine gegenüberliegende zweite Fläche (3) hat und Fluorophore (4) aufweist, wobei das Verfahren Folgendes aufweist:

- einen ersten Schritt, in welchem aufeinanderfolgend die erste Fläche (2) des Gegenstands (1) ausgehend von einer Mehrzahl an Beleuchtungspunkten mit einem Licht zur Anregung ($\lambda$ex) der Fluorophore (4) beleuchtet (F1) wird, wobei der erste Beleuchtungsschritt eine Abfolge von Beleuchtungsphasen ausgehend von einem Beleuchtungspunkt umfasst, welcher der jeweiligen Phase zugeordnet ist, und
- einen ersten Schritt, in welchem nacheinander eine erste Reihe von Bildern aufgezeichnet (F2) wird, wobei ein Bild dieser Reihe, in jeder der Beleuchtungsphasen der ersten Fläche, mittels eines Detektors erstellt wird, der eine Mehrzahl an Detektionspunkten aufweist, welche gleichzeitig ein Licht ($\lambda$ex, $\lambda$em) detektieren, das von der zweiten Fläche (3) des Gegenstands (1) ausgesendet wird,
wobei das Verfahren derart beschaffen ist, dass die Dichte der Beleuchtungspunkte geringer als die Dichte der Detektionspunkte ist, wobei das Verfahren anschließend Folgendes aufweist:

- einen zweiten Schritt, in welchem nacheinander die zweite Fläche (3) des Gegenstands (1) mit einem Licht zur Anregung ($\lambda$ex) der Fluorophore (4) beleuchtet (F3) wird,
- einen zweiten Schritt, in welchem aufeinanderfolgend eine zweite Reihe von Bildern aufgezeichnet (F4) wird, indem ein Licht ($\lambda$ex, $\lambda$em) detektiert wird, welches von der ersten Fläche (2) des Gegenstands (1) ausgesendet wird, und
- Rekonstruieren (F5) des dreidimensionalen Bildes der Verteilung der Fluorophore (4) in dem Gegenstand (1) anhand der ersten und der zweiten Reihe von Bildern, wobei die Rekonstruktion (F5) erfolgt, indem zwei Systeme linearer Gleichungen erstellt werden, die erhalten wurden, indem zwei Sätze von Matrizes der Greenschen Funktionen verwendet wurden, welche der ersten beziehungsweise der zweiten Aufzeichnung von Bilderreihen entsprechen, wobei die beiden Systeme linearer Gleichungen kombiniert werden, um sie aufzulösen;
wobei das Verfahren **dadurch gekennzeichnet ist, dass** die Anzahl und die Verteilung der Beleuchtungspunkte und der Detektionspunkte sich zwischen dem ersten Schritt des aufeinanderfolgenden Beleuchtens und Aufzeichnens und dem zweiten Schritt des aufeinanderfolgenden Beleuchtens und Aufzeichnens unterscheidet.

**2.** Verfahren nach Anspruch 1, **dadurch gekennzeichnet, dass** das Licht, welches von der ersten Fläche 2 ausgesendet wird, und das Licht, welches von der zweiten Fläche 3 ausgesendet wird, gefiltert werden, um das Licht zur Anregung (Aex) der Fluorophore (4) auszulöschen.

**3.** Verfahren nach einem beliebigen der Ansprüche 1 und 2, **dadurch gekennzeichnet, dass** der erste und der zweite Beleuchtungsschritt (F1, F3) mit ein und derselben Lichtquelle (S) durchgeführt werden, wobei die Lichtquelle (S) und der Gegenstand (1) zwischen dem ersten und dem zweiten Beleuchtungsschritt (F1, F3) zueinander verschoben werden.

**4.** Verfahren nach einem beliebigen der Ansprüche 1 und 2, **dadurch gekennzeichnet, dass** der erste und der zweite Beleuchtungsschritt (F1, F3) mit ein und derselben Lichtquelle (S) durchgeführt werden, wobei das Anregungslicht (Aex) mittels mindestens eines Spiegels auf die erste (2) und die zweite (3) Fläche gelenkt wird.

**5.** Verfahren nach einem beliebigen der Ansprüche 1 bis 4, **dadurch gekennzeichnet, dass** der erste und der zweite Schritt des Aufzeichnens von Bilderreihen (F2, F4) mit ein und derselben Kamera (D) durchgeführt werden, wobei die Kamera (D) und der Gegenstand (1) zwischen dem ersten und dem zweiten Bildaufzeichnungsschritt (F2, F4) zueinander verschoben werden.

**6.** Verfahren nach einem beliebigen der Ansprüche 1 bis 4, **dadurch gekennzeichnet, dass** der erste und der zweite Schritt des Aufzeichnens von Bilderreihen (F2, F4) mit ein und derselben Kamera (D) durchgeführt werden, wobei das Licht, welches von den ersten (2) und den zweiten (3) Flächen stammt, mittels mindestens eines Spiegels auf die Kamera (D) gelenkt wird.

**7.** Verfahren nach einem beliebigen der Ansprüche 1 und 2, **dadurch gekennzeichnet, dass** der Gegenstand (1) zwischen dem ersten und dem zweiten Beleuchtungsschritt umgedreht wird.


**Claims**

**1.** Method for reconstructing a three-dimensional fluorescence-optical-tomography image for examining an object (1) of plate shape having a first face (2) and an opposite second face (3) and containing fluorophores (4), the method comprising:

- a first step (F1) of sequential illumination of the first face (2) of the object (1) with light ($\lambda$ex) for exciting the fluorophores (4) from a plurality of illumination points, the first illumination step comprising a succession of phases of illumination from an illumination point associated with each phase, and
- a first step of sequential acquisition of a first series of images (F2), an image of said series being formed, in each phase of illumination of the first face, by a detector comprising a plurality of detection points that simultaneously detect light ($\lambda$ex, $\lambda$em) emitted via the second face (3) of the object (1),
the method being such that the density of the illumination points is lower than the density of the detection points, the method then comprising:

- a second step (F3) of sequential illumination of the second face (3) of the object (1) with light ($\lambda$ex) for exciting the fluorophores (4),
- a second step (F4) of sequential acquisition of a second series of images by detecting light ($\lambda$ex, $\lambda$em) emitted via the first face (2) of the object (1), and
- reconstruction (F5) of the three-dimensional image of the distribution of the fluorophores (4) in the object (1) by way of the first and second series of images, said reconstruction (F5) being achieved by establishing two linear systems of equations that are established using two sets of matrices of Green's functions corresponding to the first and second acquisition of series of images, respectively, the two linear systems of equations being combined to be solved; the method being **characterized in that** the number and distribution of the illumination points and of the detection points differs between the first step of sequential illumination and acquisition and the second step of sequential illumination and acquisition.

**2.** Method according to Claim 1, **characterized in that** the light emitted via the first face 2 and the light emitted via the second face 3 are filtered to remove the light ($\lambda$ex) for exciting the fluorophores (4).

**3.** Method according to any one of Claims 1 and 2, **characterized in that** the first and second illumination steps (F1, F3) are carried out with the same light source (S), the light source (S) and the object (1) being moved relatively between the first and second illumination steps (F1, F3).

**4.** Method according to any one of Claims 1 and 2, **characterized in that** the first and second illumination steps (F1, F3) are carried out with the same light source (S), the exciting light ($\lambda$ex) being directed onto the first and second faces (2, 3) by way of at least one mirror.

**5.** Method according to any one of Claims 1 to 4, **characterized in that** the first and second steps (F2, F4) of acquisition of series of images are carried out with the same camera (D), the camera (D) and the object (1) being moved relatively between the first and second image-acquisition steps (F2, F4).

**6.** Method according to any one of Claims 1 to 4, **characterized in that** the first and second steps (F2, F4) of acquisition of series of images are carried out with the same camera (D), the light coming from the first and second faces (2, 3) being directed onto the camera (D) by way of at least one mirror.

**7.** Method according to any one of Claims 1 and 2, **characterized in that** the object (1) is flipped between the first and second illumination steps.

Figure 1 (art antérieur)

| F1 | F3 |
|---|---|
| éclairement séquentiel de la première face de l'objet | éclairement séquentiel de la seconde face de l'objet |

| F2 | F4 |
|---|---|
| acquisition d'une première série d'images | acquisition d'une seconde série d'images |

F5

Reconstruction

Figure 2

Figure 3

Figure 4

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Littérature non-brevet citée dans la description**

- **B.W.POGUE et al.** Comparison of imaging geometries for diffuse optical tomography of tissue. *Optics Express,* 1999, vol. 4 (8
- **S.R.ARRIDGE.** Optical tomography in medical imaging. *Inverse Problems,* 1999, vol. 15, R41-R93
- **V.A.MARKEL et al.** Effects of sampling and limited data in optical tomography. *Applied Physics Letters,* 2002, vol. 81 (7

- **V.A.MARKEL et al.** Dual-projection optical diffusion tomography. *Optics letters,* 2004, vol. 29 (17
- **E.E.GRAVES et al.** Singular-value analysis and optimization of experimental parameters in fluorescence molecular tomography. *J. Opt. Soc. Am.,* 2004, vol. 21 (2